# FASCICULE DE BREVET EUROPEEN

(11) **EP 1 553 933 B1**
(45) Date de publication et mention de la délivrance du brevet: **25.07.2007**
(21) Numéro de dépôt: 03776954.4
(22) Date de dépôt: 03.10.2003
(51) Int. Cl.: A61K 31/17, A61P 17/00, A61P 35/00, A61Q 19/02, A61Q 19/08, A61K 8/46

(54) **MEDICAMENT COMPRENANT UNE THIOUREE POUR SON UTILISATION EN TANT QUE DEPIGMENTANT OU AGENT ANTIMUTAGENE ET ANTICARCINOGENE**
THIOHARNSTOFF-ENTHALTENDES ARZNEIMITEL ZUR VERWENDUNG ALS DEPIGMENTIERUNGSMITTEL ODER ALS ANTIMUTAGEN UND ANTIKARZINOGEN
MEDICINE COMPRISING A THIOUREA FOR USE AS DEPIGMENTING AGENT OR ANTI-MUTAGENIC AND ANTI-CARCINOGENIC AGENT

(30) Priorité: 11.10.2002 FR 0212697
(43) Date de publication de la demande: 20.07.2005
(73) Titulaire: Lmd, 63960 Veyre-Monton (FR)
(72) Inventeur: JEAN, Daniel, F-63270 Vic-Le-Comte (FR); RABHI, Chérif, F-63110 Beaumont (FR)
(74) Mandataire: Texier, Christian
(86) Numéro de dépôt international: PCT/FR2003/002907
(87) Numéro de publication internationale: WO 2004/032912

(56) Documents cités:
- WO-A-01/64206
- WO-A-94/19948
- WO-A-02/058664
- JIN, YI ET AL: "Thermal Degradation of Sulforaphane in Aqueous Solution" JOURNAL OF AGRICULTURAL AND FOOD CHEMISTRY (1999), 47(8), 3121-3123, XP001159126 cité dans la demande
- BACHELARD, H. S. ET AL: "Studies on endemic goiter. III. An investigation of the antithyroid activities of isothiocyanates and derivatives with observations on fractions of milk from goitrous areas" AUSTRALIAN JOURNAL OF BIOLOGICAL SCIENCES (1963), 16, 177-91, XP008018337

## Description

La présente invention concerne des médicaments contenant des thiourées ou leurs dérivés mono ou dioxydés, en particulier ayant une action dépigmentante, mais également en tant qu'agent antimutagène et/ou anticarcinogène.

La pigmentation de la peau chez les humains provient d'une série complexe de processus cellulaires qui s'effectue dans une unique population de cellules appelées mélanocytes. Les mélanocytes sont situés dans la partie inférieure de l'épiderme, et leur fonction est de synthétiser un pigment brun, la mélanine, qui protège le corps des effets dommageables des radiations ultra violettes. La mélanine est déposée dans les mélanosomes, vésicules présentes à l'intérieur des mélanocytes. Les mélanosomes sont expulsés des mélanocytes et véhiculés vers la surface de la peau par les kératinocytes, qui assimilent la mélanine contenue dans les mélanosomes. La teinte foncée de la peau est proportionnelle à la quantité de mélanine synthétisée par les mélanocytes et transférée aux kératinocytes. Dans certains cas, il est préférable de réduire ou d'inhiber la mélanogénèse, par exemple, pour éclaircir la peau, pour éliminer les taches de vieillesse ou pour réduire l'hyperactivité des mélanocytes.

Pendant longtemps, les compositions cosmétiques contenant un péroxyde tel que le péroxyde d'hydrogène ou le péroxyde de zinc ont été utilisées dans le but d'enlever les taches, telles que les taches de rousseur, qui apparaissent sur la peau. Toutefois, les péroxydes sont extrêmement instables et, en conséquence, leur stockage est problématique. De plus, l'incorporation stable de ces peroxydes dans des bases cosmétiques est difficile et les péroxydes eux-mêmes n'ont pas un effet suffisamment blanchissant.

D'un autre côté, des préparations cosmétiques comprenant de la vitamine C, de la cystéine ou du soufre colloïdal ont commencé à être utilisées dans le but de blanchir la peau. Toutefois, les effets de ces substances ne sont pas satisfaisants.

Pendant longtemps, l'hydroquinone a été la molécule dépigmentante de référence et employée dans de nombreuses préparations de dermo-cosmétique. Toutefois, ce produit n'est pas sans danger et présente une cytotoxicité importante pour les mélanocytes susceptibles de provoquer des dépigmentations irréversibles.

Récemment, l'acide kojique a été utilisé efficacement en tant que substance inhibant la formation de la mélanine dans la peau humaine. En conséquence, différentes préparations cosmétiques destinées à dépigmenter la peau et contenant de l'acide kojique (publication de brevet japonais n°56-18569) ou un ester de l'acide kojique avec un acide carboxylique aromatique telle que l'acide cinnamique ou l'acide benzoïque (publication de brevet japonais N° 60/100005) ou des diester de l'acide kojique (publications des brevets japonais N°61-60801 et 60-17961) ont été décrites. Ces acides kojiques et esters d'acide kojique sont donc connus comme étant des substances capables d'inhiber la mélanogénèse. Toutefois, l'acide kojique présente une efficacité variable selon les individus et en moyenne insuffisante.

Les isothiocyanates et thiocyanates ont également été décrits comme dépigmentant (WO 02/058664).

En conséquence, la recherche d'autres produits dépigmentants est toujours d'actualité.

Jusqu'à présent, seules les phénylthiourées ont été décrites comme possédant une activité dépigmentante (demande de brevet n° US 2002/0044914). Certaines autres thiourées comme la thiourée, la phénylthiourée et la diméthylthiourée sont connues pour être sensibilisantes (Contact and photocontact sensitivity problems assiociated with thiourea and its derivatives : A review of the literature and case reports. A. Dooms-Goosens et al., British Journal of Dermatology, 116, 4, 573-579).
Par ailleurs, certaines d'entre elles comme le carbimazole, sont utilisées comme anti-tyroïdes.

De façon surprenante, les déposants ont découvert que certaines molécules appartenant à la famille des thiourée avaient un effet inhibiteur très net de la synthèse de la mélanine in vitro.

Certes, la 1,3-bis-(5-méthanesulfinylbutyl) thiourée est connue comme étant un des produits de dégradation du sulforaphane (isothiocyanate ayant une activité dépigmentante : WO 02/058664) lors d'un contact prolongé avec l'eau, et en particulier avec de l'eau chaude par le procédé suivant : 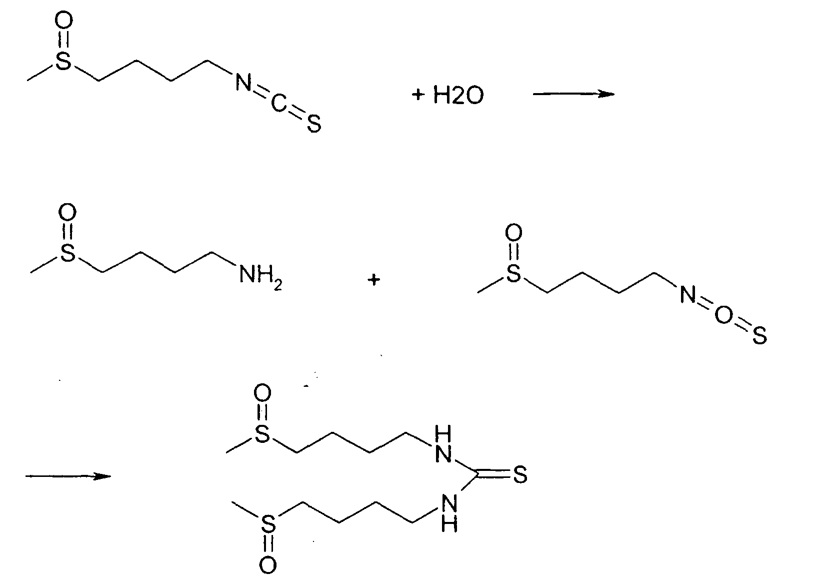 (Thermal degradation of sulforaphane in aqueous solution, Yi Jin, Mingfu Wang, Robert T. Rosen, and Chi-Tang-Ho. J. Agric. Food Chem. 1999, 47, 3121-3123). Toutefois, son activité pharmaceutique ou cosmétique, en particulier en tant que dépigmentant n'a jamais été décrit ou suggéré dans les documents de l'art antérieur, et il n'était pas évident qu'un produit de dégradation d'un produit dépigmentant ait une activité supérieure audit produit dépigmentant.

La mutagénèse se produit dans l'ADN et dans le développement des cellules spontanément ou naturellement, ou en tant qu'effet secondaire dû à des produits chimiques, à des radiations de haute énergie, au stress, etc...

Les mutagènes sont des agents qui causent de telles mutations. Ils sont aussi également souvent carcinogènes (c'est-à-dire capable d'induire le cancer). La société actuelle est maintenant au courant et concernée par la présence de mutagènes dans son environnement. Les mutagènes sont omniprésents ; certains sont naturellement présents dans les plantes, beaucoup d'autres sont produits par la combustion de matériaux organiques (en particulier durant la cuisson), et d'autres sont produits par l'industrie. De nos jours, la population se retrouve confrontée à beaucoup de problèmes de santé. Beaucoup de ces problèmes de santé sont le résultat des lésions des cellules et de l'ADN du corps humain causés par les mutagènes et mais aussi par des facteurs de mutagénèse, facteurs qui contribuent à augmenter les lésions des cellules et de l'ADN. Ces mutagènes et facteurs de mutagénèse incluent entre autres la pollution, le stress, le vieillissement, la fumée de cigarette, la lumière ultraviolette, l'exercice excessif, les lésions des tissus, etc.... Parmi les maladies résultant des lésions des cellules et de l'ADN, on peut citer : le vieillissement, les taches de vieillesse, les cancers, la cataracte, la peau sèche, la fatigue, les cancers de la peau, les dommages causés par le stress et les rides.

Bien que des compositions pharmaceutiques existent pour induire une activité antimutagène à l'intérieur du corps, elles possèdent parfois des effets secondaires significatifs.

En conséquence, un besoin existe de trouver un nouveau produit ayant un effet antimutagène pour prévenir la mutagénèse à l'intérieur du corps et un effet anticarcinogène.

De façon surprenante, les déposants ont découvert que certaines molécules appartenant à la famille des thiourées avaient un effet antimutagène et anticarcinogène très net aussi bien vis-à-vis des substances mutagènes que vis-à-vis des UVB.

La présente invention concerne donc un médicament comprenant au moins une thiourée de formule générale I suivante : 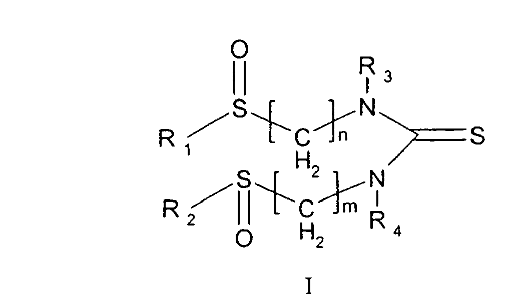 dans laquelle :
n est un nombre entier compris entre 1 et 12,
m est un nombre entier compris entre 1 et 12,
R₁, R₂, R₃ et R₄ représentent indépendamment l'un de l'autre un atome d'hydrogène, un groupe alkyle en C₁-C₆ ou un groupe aryle,
ou au moins un de ses dérivés mono ou dioxydés de formules générales IIa, IIb et III suivantes : 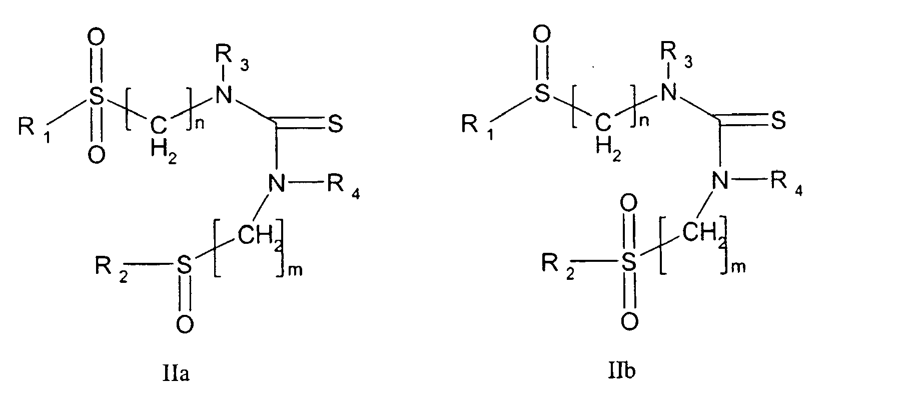 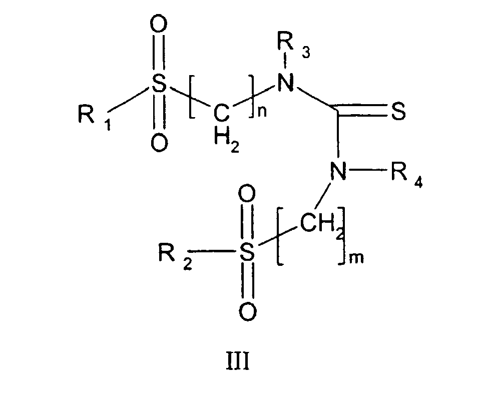 dans lesquels les R₁, R₂, R₃, R₄, m et n sont tels que définis ci-dessus,
ou leurs mélanges.

Par le terme « groupe alkyle en C₁-C₆», on entend au sens de la présente invention tout groupe alkyle de 1 à 6 atomes de carbones, linéaire ou ramifié, en particulier, le groupe CH₃.

Par le terme « groupe aryle », on entend au sens de la présente invention un ou plusieurs cycles aromatiques ayant 5 à 8 atomes de carbones, pouvant être accolés ou fusionnés. En particulier, les groupes aryles peuvent être des groupes phényle ou naphtyle et peuvent être substitués par des atomes d'halogène, des groupes alkyle tels que définis ci-dessus, le groupe OH ou le groupe nitro.

Avantageusement les thiourées de formules générales I, IIa, IIb et III, sont telles que les groupes R₁ et R₂ sont identiques, R₃ et R₄ sont identiques et m = n.
De façon encore plus avantageuse, R₁ = R₂ = CH₃.
Avantageusement m = n = 4.
De façon avantageuse R₃ = R₄ = H.
De façon encore plus avantageuse, il s'agit de la 1,3-bis-(5-méthanesulfinylbutyl) thiourée, de la 1-(5-méthanesulfinylbutyl)-3-(5-méthanesulfonylbutyl) thiourée, ou de la 1,3-bis-(5-méthanesulfonylbutyl) thiourée de formules suivantes : 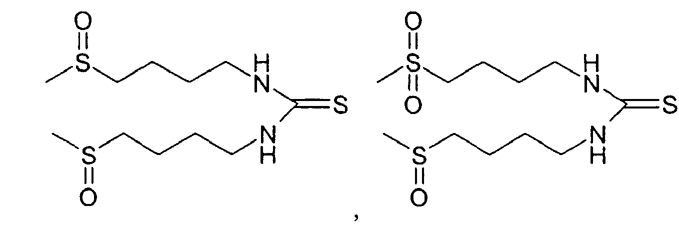 et

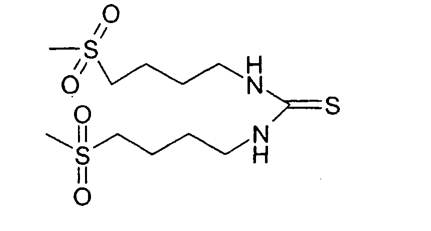

Les thiourées selon la présente invention sont soit disponibles commercialement, soit peuvent être préparés, par exemple pour la 1,3-bis(5-méthanesulfinylbutyl)-thiourée, par dégradation thermique du sulforaphane. En particulier les dérivés mono ou dioxydés peuvent être obtenus à partir de la thiourée non oxydée correspondante par action d'un agent oxydant tel que par exemple le péroxyde d'hydrogène.

De façon avantageuse, le médicament selon la présente invention est utile pour inhiber la tyrosinase, en particulier réduire de 50 % l'action de la tyrosinase, inhiber la synthèse de la mélanine, réduire l'hyperactivité des mélanocytes.

De façon avantageuse, le médicament selon la présente invention est utile en tant qu'agent antimutagène, en particulier, vis-à-vis des substances mutagènes et/ou vis-à-vis des UVB, et/ou en tant qu'agent anticarcinogène.

Avantageusement, ce médicament peut prévenir l'apparition de cancers, en particulier de cancers de la peau, des taches de vieillesse, du vieillissement, notamment celui de la peau, et des rides.

La présente invention concerne également des compositions cosmétiques comprenant au moins une thiourée, selon la présente invention, de formule générale 1 suivante : 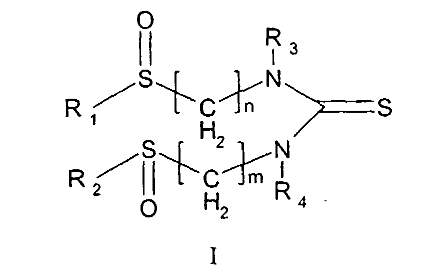 dans laquelle:
n est un nombre entier compris entre 1 et 12,
m est un nombre entier compris entre 1 et 12,
R₁, R₂, R₃ et R₄ représentent indépendamment l'un de l'autre un atome d'hydrogène, un groupe alkyle en C₁-C₆ ou un groupe aryle,
ou au moins un de ses dérivés mono ou dioxydés de formules générales IIa, IIb et III suivantes : 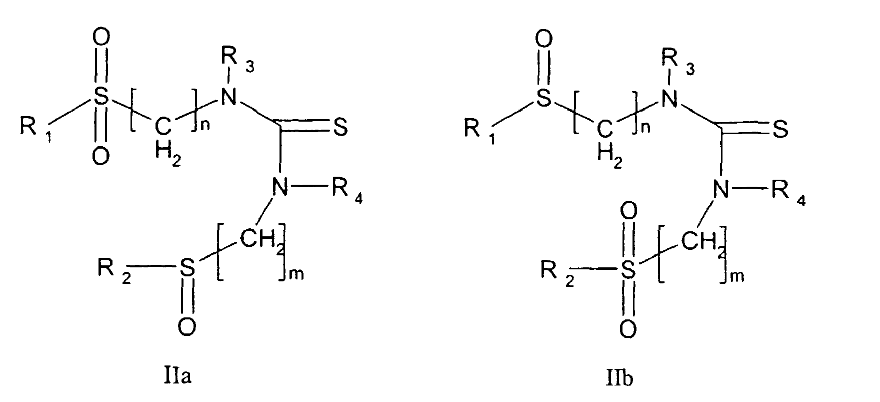 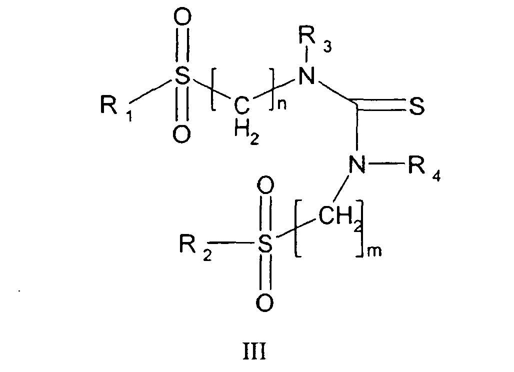 dans lesquels les R₁, R₂, R₃, R₄, m et n sont tels que définis ci-dessus.
ou leurs mélanges.

Avantageusement cette composition cosmétique est une composition dépigmentante. Elle peut être utilisée pour éclaircir, blanchir ou dépigmenter l'épiderme, éliminer les taches de la peau, en particulier de vieillesse ou de rousseur, ou prévenir la pigmentation de l'épiderme. De façon avantageuse, cette composition est destinée à une administration par voie topique.

De façon avantageuse, le médicament ou la composition cosmétique selon la présente invention se trouve sous une forme à usage orale ou topique, avantageusement à usage topique.
Il ou elle pourra se présenter sous les formes qui sont habituellement connues pour ce type d'administration, c'est à dire notamment les lotions, les mousses, les gels, les dispersions, les sprays, les sérums, les masques, les laits corporels ou les crèmes par exemple, avec des excipients permettant notamment une pénétration cutanée afin d'améliorer les propriétés et l'accessibilité du principe actif. Ces compositions contiennent généralement, outre le médicament ou l'actif cosmétique selon la présente invention, un milieu physiologiquement acceptable, en général à base d'eau ou de solvant, par exemple des alcools, des éthers ou des glycols. Elles peuvent également contenir des agents tensioactifs, des conservateurs, des agents stabilisants, des émulsifiants, des épaississants, d'autres principes actifs conduisant à un effet complémentaire ou éventuellement synergique, des oligo-éléments, des huiles essentielles, des parfums, des colorants, du collagène, des filtres chimiques ou minéraux, des agents hydratants ou des eaux thermales.

La présente invention concerne également un traitement cosmétique de la peau par application sur la peau d'une composition cosmétique selon la présente invention.

Les exemples suivant sont donnés à titre indicatif.

### Exemple 1: Synthèse de la 1,3-bis-(5-méthanesulfinylbutyl)-thiourée à partir du sulforaphane

### Synthèse du (D,L)-sulforaphane :

On dissout 40 g de 4-chlorobutyronitrile (réf. Aldrich C 3,000-0) dans 800 ml d'alcool éthylique absolu préalablement distillé sur sodium.
On ajoute ensuite 27 g de méthane thioate (réf. Fluka 71742) et on laisse sous agitation à 25°C pendant 15 heures. La suspension est filtrée sur papier et évaporée sous pression réduite. On reprend par 400 ml d'éther éthylique. On filtre à nouveau sur papier. On obtient une solution éthérée contenant 32 g de 4-méthylthiobutyronitrile brut.
On prépare une suspension de 25 g d'hydrure de lithium-aluminium dans 400 ml d'éther éthylique.
On ajoute progressivement la solution de 4-méthylthiobutyronitrile à la suspension d'hydrure de lithium-aluminium, puis on porte à reflux pendant 2 h 30.
La suspension est ensuite neutralisée en ajoutant lentement et sous reflux 80 ml d'eau distillée. Quand l'ébullition cesse, on ajoute ensuite 120 ml d'eau distillée pour achever la neutralisation de l'hydrure restant. On filtre sur verre fritté. L'insoluble est lavé sur le filtre par 200 ml d'éther éthylique. Les fractions éthérées sont réunies et évaporées à sec. On obtient 26,9 g de méthylthiobutylamine. On reprend le produit obtenu par 80 ml d'acétone à laquelle on ajoute petit à petit 23 ml de peroxyde d'hydrogène à 35 %. On place une nuit au bain-marie à 50°C.
On ajoute ensuite un peu de charbon actif, on filtre et on ajoute lentement 200 ml de chloroforme contenant 20 ml de thiophosgène, puis 300 ml d'une solution aqueuse d'hydroxyde de sodium à 5 %. On laisse agir 30 min.
On extrait ensuite le mélange à contre-courant par 8 fois 200 ml de dichlorométhane. La phase organique est recueillie, séchées sur sulfate de sodium et évaporée.
Le résidu est ensuite rectifié à 135 °C sous 7.10⁻² Torr. On obtient 12,5 g de D,L-sulforaphane dont l'identité est vérifiée par spectrométrie de masse.

### Synthèse de la 1,3-bis-(5-méthanesulfinylbutyl)-thiourée :

Les spectres infrarouges ont été obtenus sur un appareil Perkin-Elmer 1600 F11R (neat).
Les spectres de RMN du proton et du carbone 13 ont été obtenus sur un appareil Brucker AM 200 SY à 200 MHz pour le proton, 50,3 MHz pour le carbone. Les déplacements chimiques sont indiqués en partie par million (ppm) par rapport au signal du chloroforme deutérié CDC13 à 7,25 ppm pour le proton et 76,9 ppm (raie centrale du deutériochloroforme) pour le carbone.
Les spectres de masse ont été obtenus sur un appareil Normag/SIDAR V 2.3 par les techniques d'ionisation chimique (NH₃) ou d'impact électronique.

### Chromatographie

Les réactions ont été suivies en chromatographie sur couche mince sur des plaques de type silice gel 60F 254 (Merck, Art. 7735). Réactifs et produits ont été visualisés en lumière UV puis par traitement avec une solution éthanolique à 10% d'acide phosphomolybdique suivi d'un chauffage.
Les Flash chromatographies ont été effectuées sur gel de silice 1CN 60, 230-400 mesh.

### Distillation des solvants

L'éther éthylique et le THF ont été distillés sous azote sur sodium/benzophénone. Le cyclohexane, l'acétate d'éthyle et le méthanol utilisés pour les chromatographies ont été distillés avant emploi.

### Méthode

Sous atmosphère inerte, 2g (11 mmol) de sulforaphane obtenu ci-dessus sont dilués dans 15 ml d'eau, puis la solution est portée à reflux pendant 24 heures, à l'abri de la lumière.
A température ambiante, la solution est concentrée sous pression réduite et le résidu est repris par du dichlorométhane pour être chromatographié sur une colonne de gel de silice (éluant : CH₂Cl₂/CH₃OH : 90 /10) pour conduire à 1,70 g (5 mmol) de 1,3-bis-(5-méthanesulfinylbutyl)-thiourée (bis MSiBT) sous la forme d'une huile incolore, avec un rendement de 43%.
**RMN ¹H (300 MHz)** : 6,45 (dl, 2H, NH), 3,53 (t. H, J = 6,0 Hz), 2.87 (m, 4H), 2,65 (s, 6H), 1,78 (m, 8H).
**RMN ¹³C (50,3 MHz) :** 24,0 (CH₂), 32,1 (CH₂), 41,2 (CH₂-CH₂), 47,2 (CH₃), 57,4 (CH₂SO), C (IV) non observé.
**IR (ν, cm-1)** : 2176, 2096,1140,940 cm⁻¹.
**IC.MS** *m*/*z* : 312 (MH⁺).

### Exemple 2 : Préparation de la 1-(5-méthanesulfinylbutyl)-3-(5-méthanesulfonylbutyl)-thiourée

L'oxydation d'un seul des deux atomes de soufre de la molécule de sulforaphane est obtenue en ne réalisant pas la réaction de l'exemple 1 de synthèse de la 1,3-bis-(5-méthanesulfinylbutyl)-thiourée sous atmosphère inerte mais à l'air libre.
1 g (5,5 mmol) de sulforaphane sont dilués dans 10 ml d'eau, puis la solution est portée aux reflux pendant 24 heures, à l'abri de la lumière.
A température ambiante, la solution est concentrée sous pression réduite et le résidu est repris par du dichlorométhane pour être chromatographié sur une colonne de gel de silice (éluant : CH₂Cl₂ / CH₃OH : 90/10) pour conduire à 200mg de 1-(5-méthanesulfinylbutyl)-3-(5-méthanesulfonylbutyl)-thiourée (MSBMSBT) sous la forme d'une huile incolore, avec un rendement de 12%.
**RMN ¹H (300 MHz) :** 6,55 (sl, 2H, NH), 3,55 (t, 4H, CH₂), 3,15 (t, 2H, SO₂CH₂), 2,80 (t, 2H, SOCH₂), 2,60 (s, 3H, SOCH₃), 2,90 (s, 3H, SO₂CH₃), 1,80-1,87 (m, 8H, CH₂).
**RMN ¹³C (50,3 MHz) :** 20,5 (CH₂), 28,5 (CH₂), 39,8 (CH₃SO), 41,0 (CH₃SO₂), 43,5 (CH₂-NH), 54,1 (CH₂SO), 44,8 (CH₂SO₂), 183,35 (C=S).
**IR (v, cm-1) :** 2176, 2096, 1140, 940 cm⁻¹.
**IC.MS *m*/*z* :** 328 (MH⁺).

### Exemple 3 : préparation de la 1,3-bis-(5-méthanesulfonylbutyl) thiourée à partir de 1-isothiocyanato-4- méthylsulfonylbutane

1 g, (5 mmol) d'une solution de 1-isothiocyanato-4-méthylsulfonylbutane dans 15 ml d'eau est porté à reflux pendant 3 heures. A température ambiante, la solution est concentrée sous pression réduite et le résidu purifié par recristallisation au méthanol pour conduire à 535 mg de la 1,3-bis-(5-méthanesulfonylbutyl) thiourée (bis MSoBT) sous la forme d'un solide beige, soit un rendement de 40%.
**RMN ¹H (300 MHz) :** 6,50 (sl, H, NH), 3,55 (t, 4H, J = 6.0 Hz), 2,75 (t, 4H, SO₂CH₂), 2,60 (s. 6H, SO₂CH₃), 1,70-1,90 (m, 8H, CH₂CH₂).
**RMN ¹³C (50,3 MHz) :** 21,0 (CH₂), 29,2 (CH₂), 38,5 (CH₃), 44,0 (CH₂-NH), 53,9 (CH₂SO), 182,6 (C=S).
**IR (v, cm-1) :** 2168, 2096, 1144, 930 cm⁻¹.
**IC.MS *m*/*z*** : 344 (MH⁺).
Point de fusion: 142 °C.

### Exemple 4: préparation de la bis (MSoBT) à partir de la bis MSiBT

A une solution de bis MSiBT (1 g, 3 mmol) dans 10 ml d'acétone sont ajoutés lentement à O°C 0,70 ml (2,2 équiv., 6,6 mmol) de péroxyde d"hydrogène à 35%. Le milieu réactionnel est alors laissé évoluer à température ambiante pendant 40 heures. L'acétone est éliminée par évaporation sous vide. Le résidu aqueux est repris dans du chloroforme et la phase est directement piégée par du sulfate de sodium. La phase organique est concentrée sous vide et le résidu purifié par recristallisation au méthanol pour conduire à la bis MSoBT sous forme d'un solide beige avec un rendement de 30%.

### Exemple 5: étude de l'effet anti-tyrosinase de la bis MSoBT en comparaison avec l'acide kojique

### Mesure du pouvoir inhibiteur de la tyrosinase :

On utilise la réaction suivante : la L Dopa (L-3,4-dihydroxyphenylalanine, obtenue chez la société Sigma (ref D-9626)) incolore est oxydée en dopachrome colorée absorbant à 475 nm. Cette réaction est catalysée par la tyrosinase fongique (EC 1.14.18.1, obtenue chez la société Sigma (réf T-7755)). La cinétique de la réaction est enregistrée par la mesure de la densité optique (D.O.) en fonction du temps à 30° C.

Les compositions des différentes solutions utilisées sont les suivantes :

### Tampon pH 6,8 :

Tampon phosphate 0,1M, pH 6,8
Solution de substrat :
5mM de L-DOPA dans la solution tampon pH 6,8

### Solutions d'inhibiteurs :

Les molécules inhibitrices sont dissoutes directement dans le tampon pH 6,8, dans le méthanol à 50 % (méthanol - eau distillée) ou dans le méthanol pur selon leur solubilité.
Les concentrations en poids par volume des différentes solutions d'inhibiteurs sont : 0,2 %, 0,1 %, 0,05 %, 0,025 %, 0,0125 %, 0,00625 % et 0,00312 %.

### Solution d'enzyme :

250 unités de tyrosinase dans la solution tampon pH 6,8.

L'action de la tyrosinase est évaluée par la vitesse initiale de la réaction mesurée sur les enregistrements de D.O.
On porte sur une courbe les vitesses initiales des réactions sans inhibiteurs (concentration 0) et les vitesses aux diverses concentrations testées.
Le pouvoir inhibiteur d'une molécule est défini comme la concentration qui réduit de 50 % l'action de la tyrosinase.
La lecture se fait pendant 3mn et le pourcentage d"inhibition est calculé (vitesse d'inhibition et plateau de saturation). La bis MSoBT fabriquée de la façon indiquée ci-dessus, inhibe l'activité enzymatique de la tyrosinase de 50 % à la concentration de 8,3 mM final. L'acide kojique (obtenu chez la société Aldrich ref. : 22,046-9) montre le même pourcentage d'inhibition à la concentration de 0,670 mM final.

La faible solubilité dans l'eau de la bis MSiBT et de la MSBMSBT n'a pas permis de tester ces molécules pour leur effet anti-tyrosinase.

### Exemple 6 : étude de l'effet dépigmentant de la bis MSiBT, de la MSBMSBT et de la bis MSoBT en comparaison avec l'acide kojique et la diméthylthiourée

Les molécules bis MSiBT, MSBMSBT et bis MSoBT ont également été testés sur des co-cultures mélanocytes - kératinocytes humains produisant de la mélanine afin de tester leur effet dépigmentant sur un système vivant représentant l'unité de mélanisation telle qu'elle existe dans la peau humaine fonctionnelle.
L'acide kojique et la diméthylthiourée ont été utilisés comme témoins positifs dans ce test.
Les épidermes pigmentés (phénotype noir / co-culture de kératinocytes et mélanocytes normaux à l'interface air - liquide) proviennent et sont cultivés selon les recommandations de MatteKcorporation, USA. Les milieux de cultures sont fournis également par ce fabricant. Les produits (acide kojique 35 mM, diméthylthiourée 2,8 mM obtenue chez la société Aldrich ref. : D 18,870-0, bis MSiBT 2,8 mM obtenu par le procédé indiqué ci-dessus, MSBMSBT 2,8 mM obtenu par le procédé indiqué ci-dessus et bis MSoBT obtenu par le procédé indiqué ci-dessus 2,8 mM) sont appliqués dans le milieu de culture pendant 21 jours.
Chaque produit est appliqué à quatre puits de culture en solution dans du milieu de culture. L'effet dépigmentant des produits est nettement visible puisque les épidermes témoins se pigmentent progressivement alors que les épidermes traités par les produits les plus actifs montrent une pigmentation nettement moins foncée. L'évaluation de l'effet dépigmentant est réalisée par extraction de la mélanine des puits de culture.
L'extraction est réalisée sur un pool de deux puits de culture qui sont homogénéisés dans 0,45 ml de sodium dodécyl sulfate (SDS) à 1 % contenant 0,05 mM d'EDTA et 10 mM de Tris Hcl (amino-2-(hydroxyniéthyl)-2-propanediol-1,3), pH 6,8. A chaque homogénat. 20 µl de protéinase K à 5 mg/ml est ajoutée.
La digestion est réalisée pendant une nuit à 45°C. On ajoute ensuite à nouveau 20 µl de protéinase K et l'incubation est poursuivie pendant 4 heures. On ajoute ensuite 50 µl de solution 0,5 M de carbonate de sodium et 10 µl d'une solution à 30% de péroxyde d'hydrogène. Les échantillons sont maintenus à 80°C pendant 30 min et refroidis. Les échantillons sont extraits par 100 µl d'un mélange chloroforme- méthanol (2 : l, v/v). Après centrifugation à 10,000 g pendant 10 min, la densité optique du surnageant est mesurée à 405 nm. Les produits sont comparés entre eux dans le tableau 1 suivant (les résultats sont exprimés en pourcentage d'inhibition de la mélanine dans les co-cultures) :

| **Produit testé** | **Concentration en mM** | **Inhibition %** |
|---|---|---|
| Acide kojique | 35 | 32 |
| Diméthylthiourée | 2,8 | 36 |
| Bis MSiBT | 2,8 | 7,5 |
| MSBMSBT | 2,8 | 18 |
| Bis MSoBT | 2,8 | 29,6 |

D'une façon surprenante, la bis MSiBT manifeste une activité dépigmentante plus faible que la MSBMSBT et surtout que la bis MSoBT dont la concentration nécessaire à l'obtention de 30% d'inhibition environ est 25 fois plus faible que pour l'acide kojique qui est la molécule de référence largement utilisée dans le commerce des produits destinés à éclaircir la peau.

Les produits selon la présente invention possèdent donc une activité dépigmentante.

### Exemple 7 : évaluation de l'effet antimutagène de la bis MsoBT

Cette propriété a été mise en évidence dans une version modifiée du test de Ames, le test Vitotox 10kit (6400000) de la société Thermolab Systems.

D'une façon générale le test Vitotox est basé sur des bactéries contenant l'opéron *lux* de *Vibrio fisheri* sous le contrôle transcriptionnel du promoteur *recN* muté (contrôlé par le système SOS de la bactérie). Après incubation des bactéries avec un produit génotoxique, le promoteur *recN* est déréprimé et l'opéron lux s'exprime : émission de lumière, émission qui est proportionnelle à la génotoxicité du produit. Certains produits agissent directement sur la production de lumière ou augmentent le métabolisme des bactéries, créant de faux positifs. Aussi, une souche bactérienne, possédant un opéron lux constitutif est utilisée en contrôle. Cette même souche est utilisée comme contrôle de cytoxicité (faux négatifs).

### Exemple 7.1. : évaluation de l'effet antimutagène de la bis MsoBT vis-à-vis du MMS (methylméthanesulfonate)

Afin de tester l'effet antimutagène vis-à-vis de molécules génotoxiques, on utilise une molécule génotoxique, le méthylméthanesulfonate (MMS) que l'on compare à la génotoxicité d'un mélange de molécules mutagènes de référence, 4-nitroquinoline oxide et benzo(a)pyrene. Le MMS est placé seul ou en mélange avec la bis MsoBT à des concentrations croissantes et on compare les émissions de lumière. On peut alors définir une concentration inhibitrice 50 de la mutagénèse.

Des cultures de *Salmonella typhimurium* TA 104 recN2-4 (pour le test de la génotoxicité) et de *Salmonella typhimurium* TA 104 pr1 (pour le test de la cytotoxicité) dans du milieu Nutrient Broth 8g/L sont réalisées puis incubées à 37°C sous agitation pendant une nuit (DO comprise entre 0,2 et 0,5 pour les bactéries pour le test de génotoxicité et entre 0,4 et 0,6 pour les bactéries pour le test de cytotoxicité). Le lendemain, les bactéries pour le test de génotoxicité sont diluées au 1/10 avec du milieu Nutrient Broth et au ½ pour les bactéries pour le test de cytotoxicité.

Une solution mère de MMS à 90 mM dans de l'H₂O est préparée et des dilutions sériées au ½ dans plaque 96 sont réalisées. Les produits génotoxiques de référence (4-nitroquinolineoxide (NQO)/benzo(a)pyrene(BAP)) sont utilisés à 0,4 ppm pour le NQO et 800 ppm pour le BAP). Le produit à tester est utilisé avec ou sans activation métabolique (fraction S9: homogénat microsomal contenant du cytochrome P450 : 2,45 mL/mL finale).
La cinétique d'émission de lumière, en fonction de la génotoxicité et de la cytotoxicité du produit, est mesurée pendant 3 heures (mesures toutes les 5 minutes) avec un Fluoroskan Ascent FL (ThermoLabsystems).
Les produits sont fournis par les sociétés Sigma - Aldrich.

### Résultats :

La bis MsoBT présente une concentration inhibitrice 50 de l'effet mutagène du MMS de 17 p.p.m sans S9 et de 20 p.p.m avec S9.

### Exemple 7.2 : évaluation de l'effet antimutagène de la bis MsoBT vis-à-vis des UV B

Afin d'évaluer l'effet antimutagène vis-à-vis des UVB, les cultures bactériennes sont irradiées pendant une durée permettant une émission moyenne de 20 unités lumineuses (RLU). On place ensuite la bis MsoBT à concentrations croissantes dans le milieu de culture avant irradiation et on détermine une concentration inhibitrice 50.

On opère comme dans l'exemple 1 mais sans MMS et en irradiant les cultures bactériennes pendant 20 s par des UVB 254 nm.

Dans ces conditions, la MsoBT présente une concentration inhibitrice 50 de l'effet mutagène induit par les UV B de 25 p.p.m.

## Revendications

1. Médicament comprenant au moins une thiourée de formule générale I suivante : 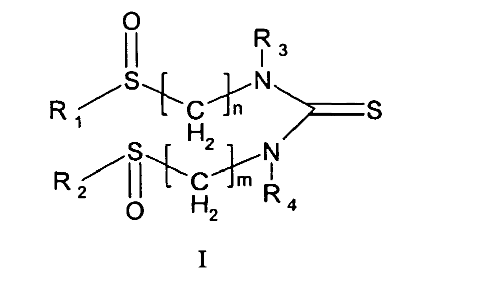 dans laquelle :
n est un nombre entier compris entre 1 et 12,
m est un nombre entier compris entre 1 et 12,
R₁, R₂, R₃ et R₄ représentent indépendamment l'un de l'autre un atome d'hydrogène, un groupe alkyle en C₁-C₆ ou un groupe aryle,
ou au moins un de ses dérivés mono ou dioxydés de formules générales IIa, IIb et III suivantes : 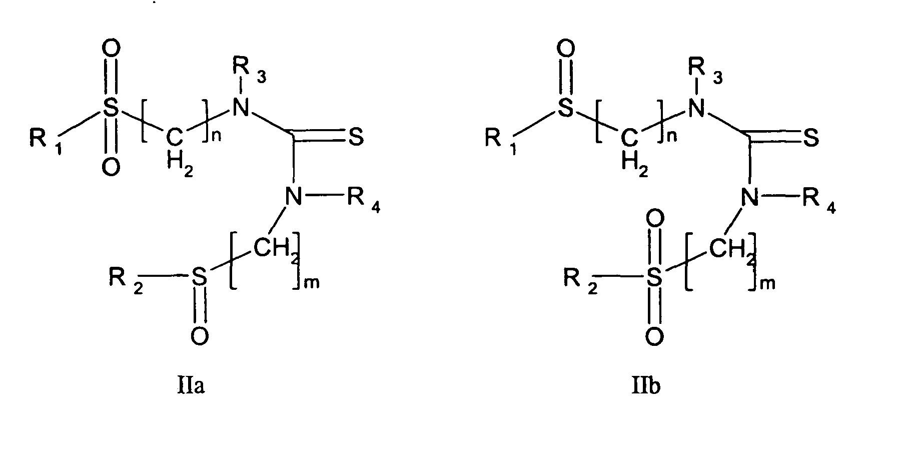 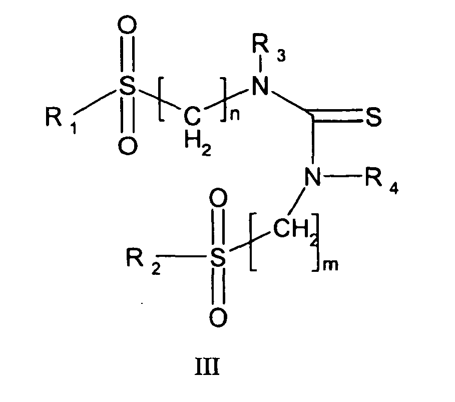 dans lesquels les R₁, R₂, R₃, R₄, m et n sont tels que définis ci-dessus.
ou leurs mélanges.

2. Médicament selon la revendication 1 pour inhiber la tyrosinase, inhiber la synthèse de la mélanine, ou réduire l'hyperactivité des mélanocytes.

3. Médicament selon la revendication 1 en tant qu'agent antimutagène et/ou anticarcinogène, avantageusement destiné au traitement et à la prévention des lésions des cellules et de l'ADN du corps humain causés par les mutagènes et par des facteurs de mutagénèse.

4. Médicament selon la revendication 3 pour prévenir l'apparition du cancer, en particulier du cancer de la peau.

5. Médicament selon l'une quelconque des revendications 1 à 3 destiné à la prévention du vieillissement, notamment celui de la peau.

6. Médicament selon l'une quelconque des revendications précédentes, **caractérisé en ce que** les groupes R₁ et R₂ sont identiques, R₃ et R₄ sont identiques et m = n.

7. Médicament selon l'une quelconque des revendications précédentes, **caractérisé en ce que** R₁ = R₂ = CH₃.

8. Médicament selon l'une quelconque des revendications précédentes, **caractérisé en ce que** et m = n = 4.

9. Médicament selon l'une quelconque des revendications précédentes, **caractérisé en ce que** R₃ = R₄ = H.

10. Médicament selon l'une quelconque des revendications précédentes **caractérisé en ce qu'**il se trouve sous une forme à usage topique.

11. Thiourée de formule générale I suivante 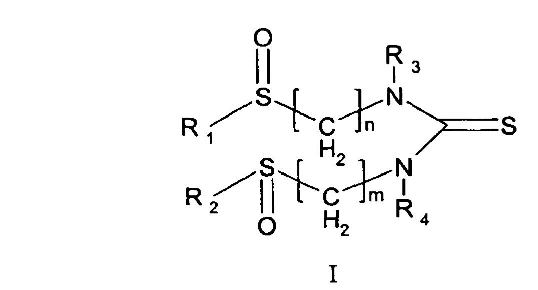 dans laquelle :
n est un nombre entier compris entre 1 et 12,
m est un nombre entier compris entre 1 et 12,
R₁, R₂, R₃ et R₄ représentent indépendamment l'un de l'autre un atome d'hydrogène, un groupe alkyle en C₁-C₆ ou un groupe aryle,
ou un de ses dérivés mono ou dioxydés de formules générales IIa, IIb et III suivantes : 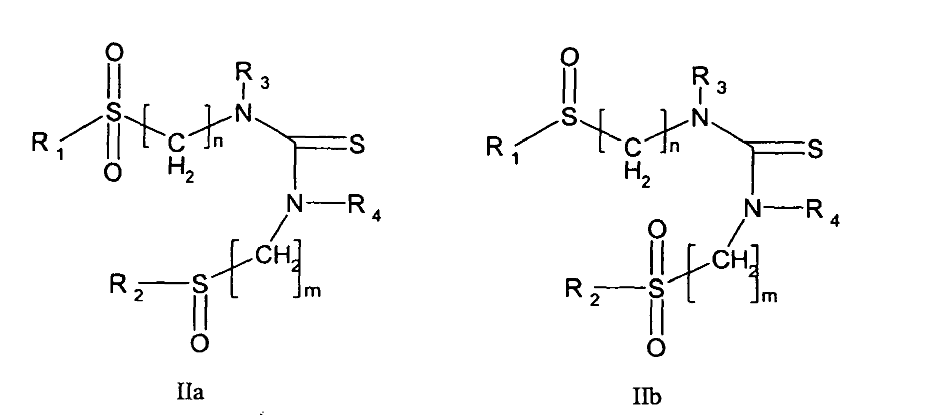 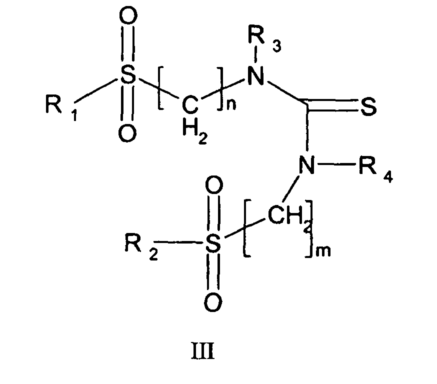 dans lesquels les R₁, R₂, R₃, R₄, m et n sont tels que définis ci-dessus,
à l'exception de la N,N-di(-méthylsulfinylbutyl) thiourée et de la dicheirolin thiourée.

12. Composition cosmétique comprenant au moins une thiourée selon la revendication 11.

13. Utilisation cosmétique d'une composition comprenant au moins une thiourée de formule générale I ou au moins un de ses dérivés mono ou dioxydés de formule génerale IIa, IIb et III tels que définis dans la revendication 1 pour éclaircir, blanchir ou dépigmenter l'épiderme, éliminer les taches de la peau, en particulier de vieillesse ou de rousseur, ou prévenir la pigmentation de l'épiderme.

14. Utilisation cosmétique d'une composition comprenant au moins une thiourée de formule générale I ou au moins un de ses dérivés mono ou dioxydés de formule générale IIa, IIb et III tels que définis dans la revendication 1 contre les rides.

## Claims

1. Medicinal product containing at least one thiourea of the following general formula I: 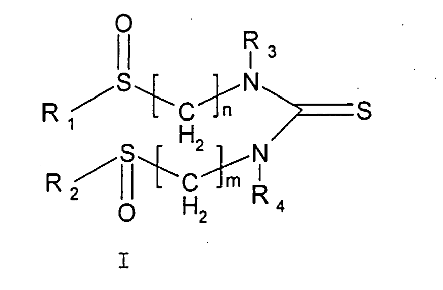 in which:
n is an integer between 1 and 12,
m is an integer between 1 and 12,
R₁, R₂, R₃ and R₄ represent, independently of one another, a hydrogen atom, a C₁-C₆ alkyl group or an aryl group,
or at least one of its monoxide or dioxide derivatives of the following general formulae IIa, IIb and III: 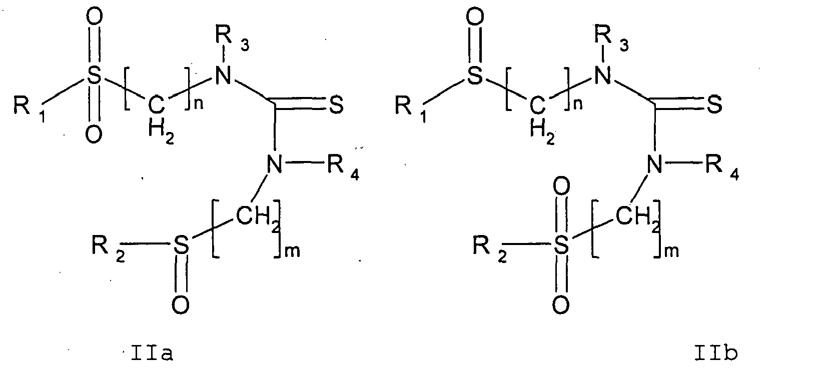 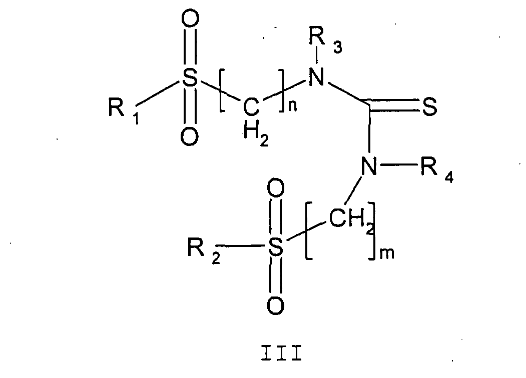
in which R₁, R₂, R₃, R₄, m and n are as defined above.
or their mixtures.

2. Medicinal product according to Claim 1 for inhibiting tyrosinase, inhibiting the synthesis of melanin, or reducing hyperactivity of melanocytes.

3. Medicinal product according to Claim 1 as antimutagenic and/or anticarcinogenic agent, advantageously intended for the treatment and for the prevention of lesions of the cells and DNA of the human body, caused by mutagens and by mutagenesis factors.

4. Medicinal product according to Claim 3 for preventing the appearance of cancer, in particular cancer of the skin.

5. Medicinal product according to any one of Claims 1 to 3, intended for the prevention of ageing, in particular that of the skin.

6. Medicinal product according to any one of the preceding claims, **characterized in that** the groups R₁ and R₂ are identical, R₃ and R₄ are identical and m = n.

7. Medicinal product according to any one of the preceding claims, **characterized in that** R₁ = R₂ = CH₃.

8. Medicinal product according to any one of the preceding claims, **characterized in that** m = n = 4.

9. Medicinal product according to any one of the preceding claims, **characterized in that** R₃ = R₄ = H.

10. Medicinal product according to any one of the preceding claims, **characterized in that** it is in a form for topical use.

11. Thiourea of the following general formula I 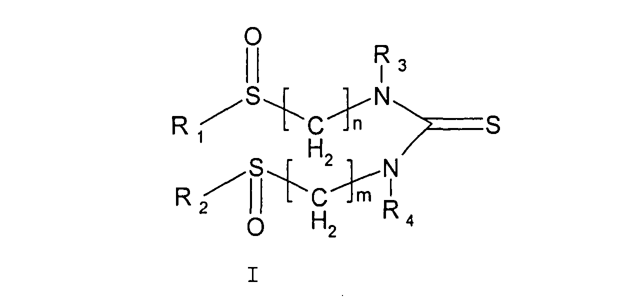 in which:
n is an integer between 1 and 12,
m is an integer between 1 and 12,
R₁, R₂, R₃ and R₄ represent, independently of one another, a hydrogen atom, a C₁-C₆ alkyl group or an aryl group,
or one of its monoxide or dioxide derivatives of the following general formulae IIa, IIb and III: 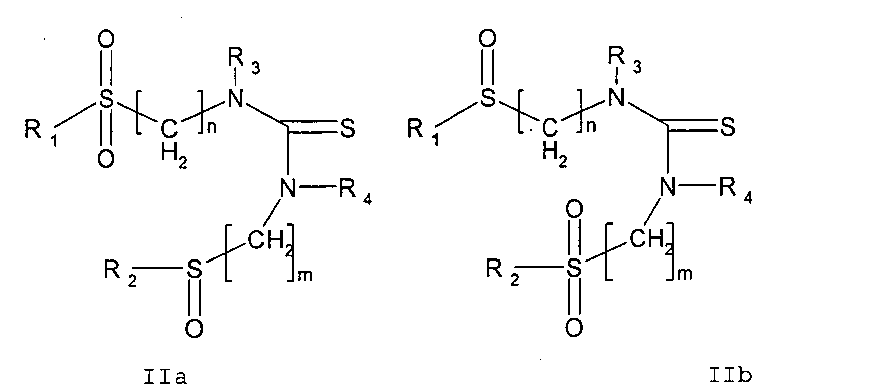 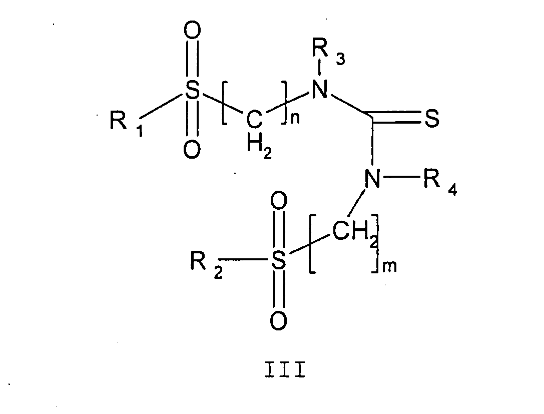
in which R₁, R₂, R₃, R₄, m and n are as defined above, with the exception of N,N-di(methylsulphinylbutyl) thiourea and dicheirolin thiourea.

12. Cosmetic composition containing at least one thiourea according to Claim 11.

13. Cosmetic use of a composition containing at least one thiourea of the general formula I or at least one of its monoxide or dioxide derivatives of the general formulae IIa, IIb and III as defined in Claim 1, for lightening, whitening or depigmenting the epidermis, removing skin blemishes, especially from ageing or freckles, or preventing pigmentation of the epidermis.

14. Cosmetic use of a composition containing at least one thiourea of the general formula I or at least one of its monoxide or dioxide derivatives of the general formulae IIa, IIb and III as defined in Claim 1, against wrinkles.

## Patentansprüche

1. Arzneimittel, umfassend wenigstens einen Thioharnstoff der folgenden allgemeinen Formel I: 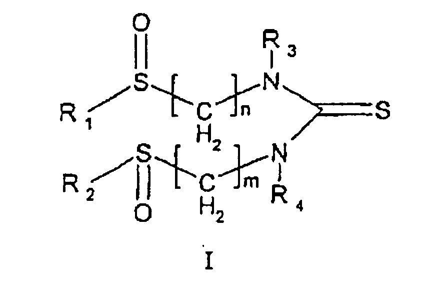 in welcher:
n eine ganze Zahl zwischen 1 und 12 eingeschlossen ist,
m eine ganze Zahl zwischen 1 und 12 eingeschlossen ist,
R₁, R₂, R₃ und R₄ unabhängig voneinander für ein Wasserstoffatom, eine C₁-C₆-Alkylgruppe oder eine Arylgruppe stehen,
oder wenigstens eines von dessen mono- oder dioxydierten Derivaten mit den folgenden allgemeinen Formeln IIa, IIb und III: 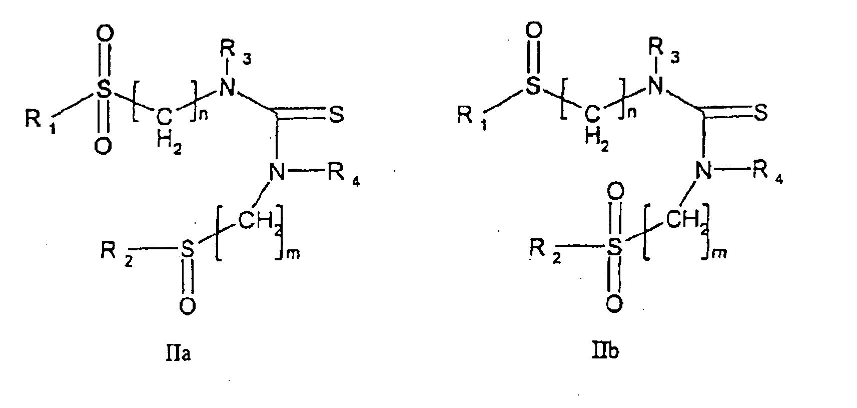 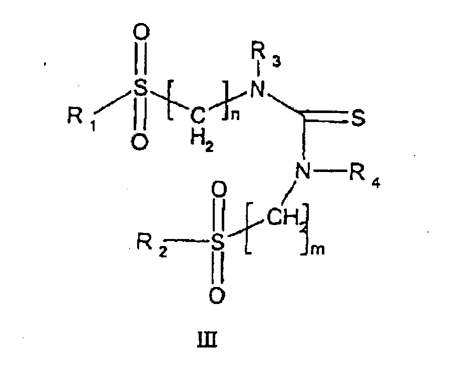
in welchen die R₁, R₂, R₃, R₄, m und n so, wie oben definiert, sind,
oder deren Mischungen.

2. Arzneimittel nach Anspruch 1, um die Tyrosinase zu hemmen, die Synthese von Melanin zu hemmen oder die Hyperaktivität der Melanozyten zu verringern.

3. Arzneimittel nach Anspruch 1 als antimutagenes und/oder antikanzerogenes Mittel, welches vorteilhafterweise für die Behandlung und die Verhütung von Läsionen von Zellen und der DNA des menschlichen Körpers, welche durch die Mutagene und durch Mutagenesefaktoren verursacht werden, bestimmt ist.

4. Arzneimittel nach Anspruch 3, um das Auftreten von Krebs, insbesondere von Hautkrebs zu verhindern.

5. Arzneimittel nach einem der Ansprüche 1 bis 3, welches für die Verhütung der Alterung, insbesondere jener der Haut bestimmt ist.

6. Arzneimittel nach einem der vorangegangenen Ansprüche, **dadurch gekennzeichnet, dass** die Gruppen R₁ und R₂ identisch sind, R₃ und R₄ identisch sind und m = n.

7. Arzneimittel nach einem der vorangegangenen Ansprüche, **dadurch gekennzeichnet, dass** R₁ = R₂ = CH₃.

8. Arzneimittel nach einem der vorangegangenen Ansprüche, **dadurch gekennzeichnet, dass** m = n = 4.

9. Arzneimittel nach einem der vorangegangenen Ansprüche, **dadurch gekennzeichnet, dass** R₃ = R₄ = H.

10. Arzneimittel nach einem der vorangegangenen Ansprüche, **dadurch gekennzeichnet, dass** es sich in einer Form für eine topische Anwendung befindet.

11. Thioharnstoff der folgenden allgemeinen Formel 1: 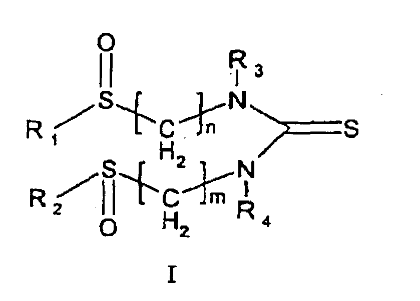 in welcher:
n eine ganze Zahl zwischen 1 und 12 eingeschlossen ist,
m eine ganze Zahl zwischen 1 und 12 eingeschlossen ist,
R₁, R₂, R₃ und R₄ unabhängig voneinander für ein Wasserstoffatom, eine C₁-C₆-Alkylgruppe oder eine Arylgruppe stehen,
oder eines von dessen mono- oder dioxydierten Derivaten mit den folgenden allgemeinen Formeln IIa, IIb und III: 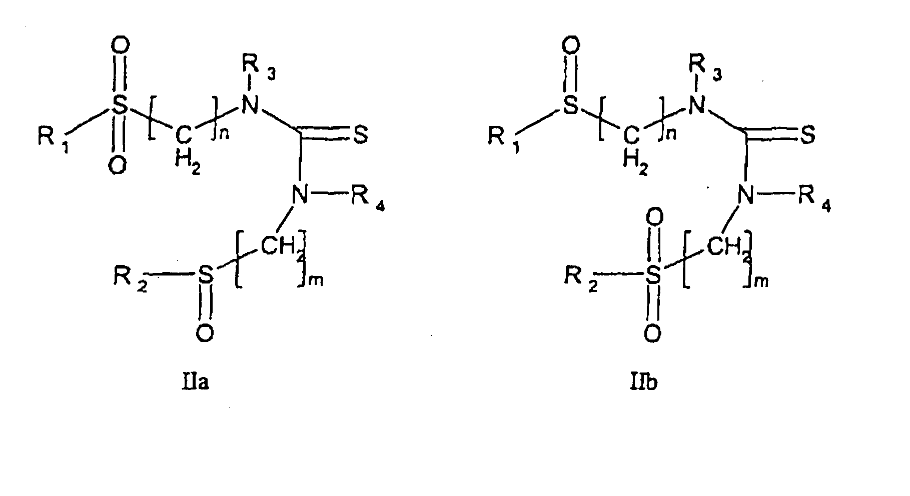 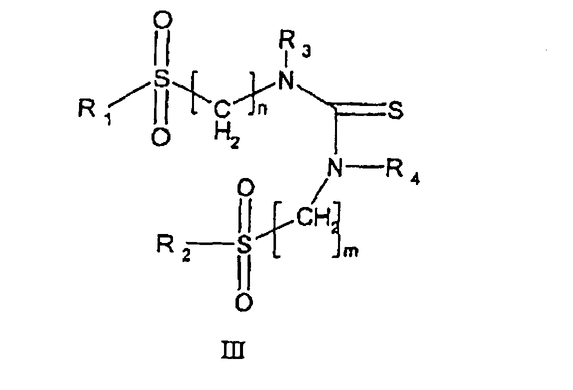
in welchen die R₁, R₂, R₃, R₄, m und n so, wie oben definiert, sind,
mit der Ausnahme von N,N-Di-(methylsulfinylbutyl)-thiohamstoff und Dicheirolinthioharnstoff.

12. Kosmetische Zusammensetzung, welche wenigstens einen Thioharnstoff gemäß Anspruch 11 umfasst.

13. Kosmetische Verwendung einer Zusammensetzung, welche wenigstens einen Thioharnstoff der allgemeinen Formel oder wenigstens eines von dessen mono- oder dioxydierten Derivaten mit der allgemeinen Formel IIa, IIb und III, wie in Anspruch 1 definiert, umfasst, um die Epidermis aufzuhellen, zu bleichen oder zu depigmentieren, Flecken der Haut, insbesondere Altersflecken oder Sommersprossen, zu entfernen oder die Pigmentierung der Epidermis zu verhüten.

14. Kosmetische Verwendung einer Zusammensetzung, welche wenigstens einen Thioharnstoff der allgemeinen Formel 1 oder wenigstens eines von dessen mono- oder dioxydierten Derivaten mit der allgemeinen Formel IIa, IIb und III, wie in Anspruch 1 definiert, umfasst, gegen Falten.
